# EUROPEAN PATENT APPLICATION

(11) **EP 3 422 225 A1**
(43) Date of publication of application: **02.01.2019**
(21) Application number: 17305807.4
(22) Date of filing: 28.06.2017
(51) Int. Cl.: G06F 19/00

(54) **MEDICAL SYSTEM AND METHOD ALLOWING A USER TO LOGIN USING A WIRELESS COMMUNICATION CONNECTION**

(71) Applicant: Fresenius Vial SAS, 38590 Brézins (FR)
(72) Inventor: GUERRINI, Alexandre, 38900 Fontaine (FR)
(74) Representative: Kusche, Robert

(57) **Abstract**

A medical system (1) comprises a host device (11) for monitoring and/or controlling an operation of at least one medical device (10) for performing a medical function. The host device (11) is configured to execute a host application (114), wherein functions of the host device (11) are accessible by a user (U) by providing login information to the host application (114). A mobile communication device (13) is configured to run a login application (134) which stores said login information, wherein the host device (11) and the mobile communication device (13) are configured to establish a wireless communication connection (D1) to transmit said login information from the mobile communication device (13) to the host device (11) to allow the user (U) to access said functions of the host device (11).

## Description

The invention relates to a medical system according to the preamble of claim 1 and to a method for operating a medical system.

A medical system of this kind comprises at least one medical device for performing a medical function, and a host device operatively connected to the at least one medical device for monitoring and/or controlling an operation of the at least one medical device. The host device is configured to execute a host application, wherein functions of the host device are accessible by a user by providing login information to the host application.

The at least one medical device may for example be an infusion device, such as an infusion pump in the shape of a volumetric (peristaltic) infusion pump or a syringe infusion pump.

Within a healthcare environment, for example within a bedroom of a patient in a ward of a hospital, a multiplicity of medical devices such as infusion devices may be placed at the bedside of a patient. Herein, the medical devices may be arranged on a rack and hence may be mechanically held on the rack in an organized fashion. The rack may serve as a communication link for communicatively connecting the medical devices for example to a hospital communication network.

Such medical devices may for example be connected to a host device in the shape of a so-called infusion manager, the infusion manager allowing to input infusion parameters for programming and initiating an infusion operation making use of a medical device in the shape of an infusion pump. The infusion manager also allows to monitor ongoing infusion operations by displaying infusion parameters and by for example also outputting alarm signals in case an alarm condition arises on one of the medical devices it is connected to.

A host device of this kind may for example be placed on a rack on which medical devices are held. A host device of this kind however may also be connected to the medical devices via a communication network, for example a hospital information network and may be placed remote form the medical devices for example in a central location in a hospital.

Generally, a user (for example a physician or a nurse) is required to log in to the host device by entering login information into a host application running on the host device. After login, the user gains access to functions of the host application, for example in order to program and start an infusion operation or to monitor ongoing infusion operations.

Such login is required on the one hand to prevent unauthorized users to gain access to the host device and to influence potentially critical infusion operations. Furthermore, from the identification of the user during login a personalized operation of the system may be provided, for example by providing recommendations for therapy assistance to the user according to its preferences. Because a user accessing the host device is identified, it may be kept track of user actions, allowing for example to provide feedback to medical staff.

Conventionally, to log in to a system, a user is required to enter a username and a password. This requires the user to have the username and password at hand. Furthermore, the user is required to type in the username and password.

There is a desire to make a login procedure as easy and comfortable for a user as possible. This also includes the setting up of a user account, which shall be easy and intuitive, yet providing for a secure login in order to prevent unauthorized third parties to gain access to the system.

WO 2014/099008 A1 discloses a technique related to tap-to-wake and tap-to-login system allowing a user of a near field device to wake up a computing platform from a deep-sleep state using a bump/tap without having to move a mouse or enter a keyboard stroke.

It is an object of the instant invention to provide a system and a method which in an easy and comfortable way allow a user to log in.

This object is achieved by means of a medical system comprising the features of claim 1.

Accordingly, a mobile communication device is configured to run a login application which stores said login information, wherein the host device and the mobile communication device are configured to establish a wireless communication connection to transmit said login information from the mobile communication device to the host device to allow the user to access said functions of the host device.

A user hence is enabled to login to the system making use of a mobile communication device. The mobile communication device runs a login application which stores login information. By establishing a communication connection between the mobile communication device and the host device the login information is transmitted from the mobile device to the host device, which processes the login information and allows the user to access functions of the host device.

Login information hence is pre-stored on the mobile communication device. The login procedure thus may take place automatically by using the mobile communication device, such that a user no longer is required to input login information, such as a username and a password, into the host device for example by typing.

Upon logging into the system, the user gains access to functions of the system. The system herein may grant access to functions according to certain privileges associated with the user, different users potentially having different privileges and hence being allowed to access different functions. In another aspect, the system may automatically configure the software according to settings associated with the user. For example, for an infusion device different configurations for example relating to a dose error reduction may exist for different wards in a hospital, such that the configuration may be adjusted depending on the ward setting the user belongs to. The system hence may be configured automatically at user authentication according to specific settings associated with the user.

For example, if the user is a doctor, the device may display a screen for a doctor with specific rights associated to and defined for a doctor's profile. If the user in contrast is a maintenance operator, the device may display a screen for maintenance, with other rights associated to and defined for a maintenance profile.

The at least one medical device may be an infusion device for performing an infusion operation, such as a volumetric (peristaltic) infusion pump or a syringe infusion pump.

The host device may be a so-called infusion manager for example connected to a rack on which medical devices are placed, the infusion manager serving to control and/or monitor infusion operations carried out by the medical devices.

The host device however could also be integrated in the medical device itself such that it forms part of the medical device, for example an infusion device. In this case the host device is formed for example by a control circuitry of the medical device, for example an infusion device, and is configured to allow a user to login to the medical device.

The mobile communication device may for example be a mobile telephone (such as a smart phone), a tablet computer, a portable laptop computer or another handheld device which is enabled to establish a wireless communication connection to the host device of the system.

The wireless communication connection may in particular be a short range wireless communication connection, such as an NFC connection, a Bluetooth connection, a Wi-Fi connection, an infrared connection or a Zigbee connection.

For example, an NFC (Near Field Communication) connection is a short-range connection making use of the NFC transmission standard for the contactless exchange of data by means of electromagnetic induction using electromagnetically coupled coils. Data transfer may be initiated by placing one device in the vicinity of the other device, a coupling being established over for example a few centimeters and at a data transmission rate of 424 kbit/s (at maximum).

In one embodiment, the host device and the mobile communication device each comprise a communication module for establishing the wireless communication connection. The communication module comprises for example an antenna for establishing a wireless connection according to for example a predefined communication standard, such as the NFC standard.

In one embodiment, the wireless communication connection is a secure connection configured for an encrypted data transfer. Login information hence is transmitted from the mobile communication device to the host device in a secure, encrypted fashion. For establishing the communication connection, for example an authentication procedure may take place in which the mobile communication device and the host device provide identification information to each other. Furthermore, encryption methods can be used to encrypt the information transfer using known encryption technologies.

In one embodiment, the login application of the mobile communication device stores, as login information, at least one of a first name of the user, a last name of the user, a login name of the user, a password of the user, a last login date, a last login method, identification information relating to the mobile communication device, and an encryption key. The first name of the user, the last name of the user, the login name of the user and the password of the user represent usual login information which a user also conventionally may have been required to input into a system for logging into the system. Furthermore, the login application may transmit information relating to a last login date and a last login method. The login application hence identifies to the host device when the user last was logged into the host device using what login method. Identification information relating to the mobile communication device may for example be a phone serial number and/or a phone ID. The login application hence also identifies the mobile communication device which the user uses to login. By transmitting an encryption key such key is exchanged in between the mobile communication device and the host device such that said encryption key may be verified and/or used for further communication.

In one embodiment, the host application stores user account information relating to the user. Such user account information is stored in the host application of the host device prior to receiving the login information from the mobile communication device. The user account information is linked to a user account and may for example include a first name of the user, a last name of the user, a login name of the user, a password of the user, a last login date, a last login method, identification information relating to the mobile communication device allowed for login, and an encryption key. Such user account information is stored in a database of the host application, wherein the host application may host a multiplicity of different user accounts to allow different users to access functions of the host device.

Upon receiving login information from the mobile communication device, the host application evaluates said login information based on the stored user account information relating to the user. For example, the host device may compare received login information to user account information of a user account. If it is found that the login information matches the user account information of a particular user account, a user is allowed to access functions of the host device.

The object is also achieved by a method to operate a medical system, in which: at least one medical device performs a medical function, and a host device operatively connected to the at least one medical device for monitoring and/or controlling an operation of the at least one medical device executes a host application, wherein functions of the host device are accessible by a user by providing login information to the host application. Herein, a mobile communication device runs a login application which stores said login information, wherein, for the user to gain access to said functions of the host device, a wireless communication connection is established between the host device and the mobile communication device to transmit said login information from the mobile communication device to the host device.

The advantages and advantageous embodiments described above for the system equally apply also to the method, such that it shall be referred to the above.

Prior to allowing a user to login to the host device, a user account must be set up in the host application of the host device. For this, in a setup phase a user may be requested to input information into the mobile communication device. Such information is stored in the login application of the mobile communication device as login information. A user account at the host device may then be created by transmitting login information relating to the user from the mobile communication device to the host device using a wireless communication connection. Upon receiving such login information, the host application stores the received information as user account information and in this way sets up the user account of the user.

Because in the setup phase no encryption key may have been exchanged in between the mobile communication device and the host device yet, the transmission of the login information from the mobile communication device to the host device during the setup phase may take place using a non-secure connection, i.e., in an unencrypted manner. Once the user account is created and during the subsequent regular operation, then, login information is transmitted from the mobile communication device to the host device using a secure connection by transferring data using common encryption technologies.

Alternatively, it is possible that already in the setup phase a secure connection is used, employing a suitable encryption technology.

The idea underlying the invention shall subsequently be described in more detail by referring to the embodiments shown in the figures. Herein:
- Fig. 1: shows a schematic drawing of a medical system;
- Fig. 2: shows a schematic configuration of a mobile communication device in connection with a host device of a medical system;
- Fig. 3A: shows a mobile communication device and a host device when creating a user account on the host device;
- Fig. 3B: shows the mobile communication device and the host device during establishment of an NFC communication connection in between the mobile communication device and the host device for transmitting login information from the mobile communication device to the host device;
- Fig. 4A: shows a mobile communication device and a host device at the outset of a login procedure;
- Fig. 4B: shows the mobile communication device and the host device during the establishment of an NFC communication connection for logging into the host device; and
- Fig. 5: shows a schematic flow diagram showing information transfer during a setup phase for establishing a user account and during a login phase for logging a user into a medical system.

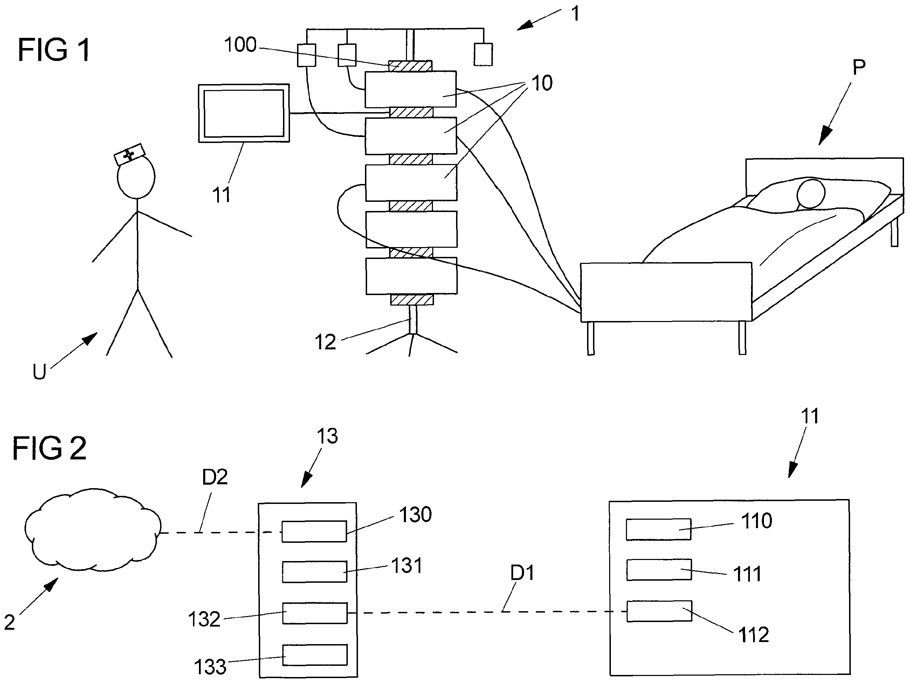

Fig. 1 shows a schematic drawing of a system 1 as it may be used in a healthcare environment, for example in a patient's bedroom in a hospital. The system 1 comprises a multiplicity of medical devices 10 for example in the shape of infusion devices such as volumetric (peristaltic) infusion pumps or syringe infusion pumps which serve to administer medical fluids such as medication or a nutritional solution for the enteral or parenteral feeding to a patient P. Medical devices 10 may for example be placed on a rack 100 which is fixed to a stand 12 such that a vertical column of medical devices 10 is formed at the bedside of the patient P, hence maintaining the medical devices 10 in an organized fashion at the bedside of the patient P.

The system 1 may further comprise a host device 11 which may serve to control and/or monitor the operation of the medical devices 10. The host device 11 may for example be constituted by an infusion manager for controlling and monitoring infusion operations carried out by medical devices 10 in the shape of infusion pumps, the infusion manager for example comprising a display and an input device such as an arrangement of pushbuttons or a keyboard for entering information and commands into the infusion manager. The host device 11 in the shape of the infusion manager may also be placed on the rack 100 and is in communication connection with the medical devices 10 such that information may be exchanged in between the host device 11 and the medical devices 10.

For performing functions of the host device 11, for example to program and start an infusion operation using a medical device 10 or to monitor ongoing infusion operations carried out by the medical devices 10, a user U may login to the host device 11. Upon logging into the system 1, the user U may for example input commands into the host device 11 and may make use of functions provided by the host device 11.

It herein is proposed to use a mobile communication device 13, as it is shown in Fig. 2 and 3A, 3B, 4A, 4B, to allow a user U to log into the system 1. The mobile communication device 13 may for example be a mobile phone such as a smart phone, a tablet computer or a laptop computer. The mobile communication device 13, as schematically shown in Fig. 2, in particular comprises a processor 133, a storage device 131, and communication modules 130, 132 for establishing data connections to an outside communication network (using the communication module 130 to establish a data connection D2) and the host device 11 (using the communication module 132 to establish a data connection D1).

The host device 11 may be a computing device such as a workstation, a personal computer or a tablet computer and in particular comprises, as schematically shown in Fig. 2, a processor 110, a storage unit 111 and a communication module 112, the communication module 112 in particular being configured to establish a communication connection D1 to the communication module 132 of the mobile communication device 13.

The communication modules 112, 132 of the host device 11 and the mobile communication device 13 may in particular be constituted to establish an NFC (Near Field Communication) communication connection D1 in between the host device 11 and the mobile communication device 13. Upon enabling an NFC functionality both on the host device 11 and the mobile communication device 13, such communication connection D1 is established once the mobile communication device 13 is brought in close proximity to the host device 11, in particular within a range of a few centimeters of an NFC antenna of the communication module 112 of the host device 11. Upon establishment of the connection D1, data may be exchanged in between the mobile communication device 13 and the host device 11 using the connection D1.

In this way a user U may login to the host device 11 by transmitting login information to the host device 11 making use of his/her mobile communication device 13. The transmission of the login information herein shall take place making use of the wireless communication connection D1 established in between the mobile communication device 13 and the host device 11.

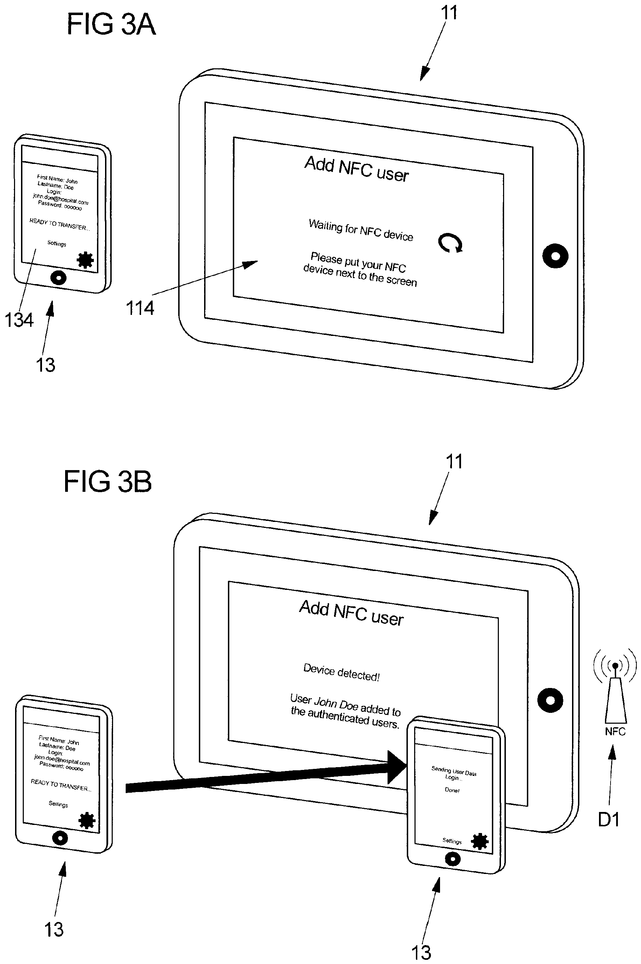

To enable a user U to login to the host device 11, a login application 134 (see Fig. 3A) is installed on the mobile communication device 13, and a host application 114 is installed on the host device 11. To enable a user U to login to the host device 11, it initially is necessary to, in a setup phase, create a user account on the host device 11, which may be carried out as it is depicted in Fig. 3A and 3B.

For creating the user account, a user U first is requested, by the login application 134 running on the mobile communication device 13, to enter login information into the mobile communication device 13, such as a first name, a last name, a login name and a password. The user U has to enter this information only once into the login application 134 at an initial setup phase, upon which the login application 134 stores the information in an internal database.

In another step during the setup phase, an administrator accesses the host device 11 and, by for example accessing the settings of the host device 11 and the host application 114, creates a new user account in the host application 114. Upon creating the user account, the communication module 112 of the host device 11 starts searching for mobile communication devices 13, and upon detecting a mobile communication device 13 brought into proximity with the communication module 112 establishes a communication connection D1 to the mobile communication device 13, as it is illustrated in Fig. 3B.

Once the connection D1 is established during the setup phase, login information for the user U is transferred from the mobile communication device 13 to the host device 11 and is stored in connection with the user account in a database in the host application 114.

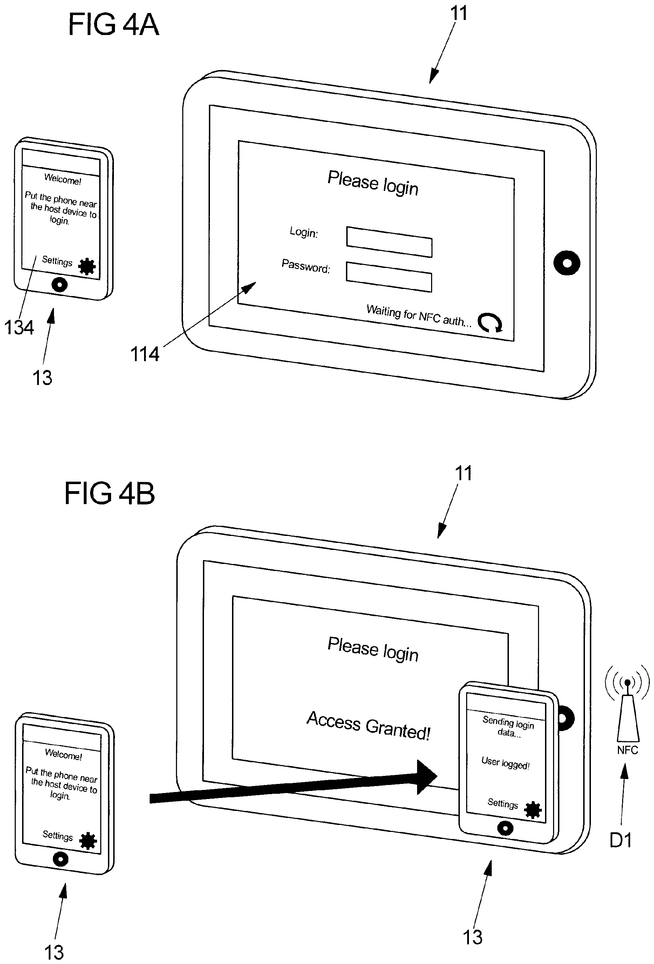

Once the user account has been created by transmitting the login information relating to the user U to the host device 11 and its host application 114, during subsequent regular operation the user U is enabled to log into the system 1 by making use of his/her mobile communication device 13. For this, as illustrated in Fig. 4A and 4B, the user U brings its mobile communication device 13 into close proximity with the communication module 112 of the host device 11 such that a wireless (NFC) data connection D1 is established in between the mobile communication device 13 and the host device 11. Upon establishment of the communication connection D1, login information stored in the login application 134 is transferred from the mobile communication device 13 to the host application 114 of the host device 11. The host application 114 compares the received login information with the pre-stored account information of the user account, and if the received information matches the information stored in the host application 114, the host application 114 concludes that the user U is authorized to access functions provided by the host device 11.

During the login procedure, login information such as the first name, last name, login name and password of the user U, may be transferred from the mobile communication device 13 to the host device 11. In addition, information relating to a last login date, a last login method, identification information relating to the mobile communication device 13 such as a serial number of the mobile communication device 13 or an identification may be transmitted from the mobile communication device 13 to the host device 11. The host device 11 may compare such transmitted information to information stored within the host device 11 in connection with the user account of the user U, such that by the comparison the user U may be authenticated.

Preferably, the exchange of information during the login procedure takes place using a secure communication connection D1. For this, data transferred from the mobile communication device 13 to the host device 11 may be encrypted using common encryption technology. An exchange of an encryption key herein may take place during the setup phase, during which the mobile communication device 13 may transmit an encryption key to the host device 11 such that both the mobile communication device 13 and the host device 11 are in possession of a common encryption key.

The exchange of information during the setup phase generally may take place in a non-secure, non-encrypted manner, because prior to the initial transfer of data for setting up the user account no encryption key may have been exchanged in between the mobile communication device 13 and the host device 11.

Alternatively, already in the setup phase encryption and hence a secure connection may be used. For example, both devices 11, 13 may share a specific encryption key stored on each device 11, 13, hidden for example in the application software.

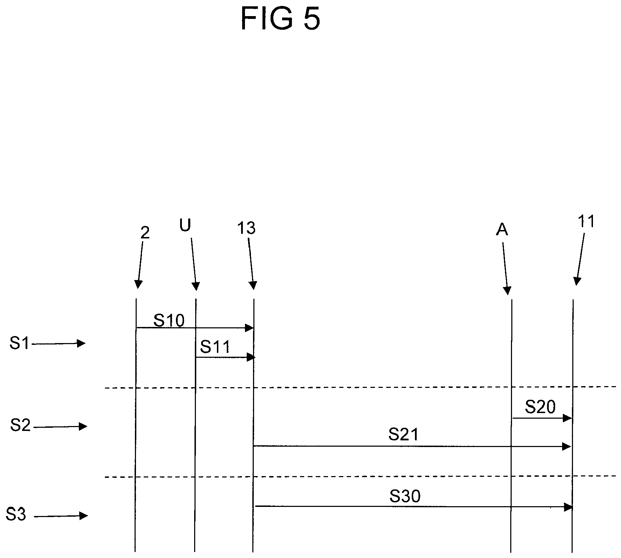

Fig. 5 shows a general flow diagram illustrating the setup phase (S1, S2) and the login phase (S3).

During the setup phase, in an initial step S1 the login application 134 is downloaded from an outside communication network 2, for example the Internet, to install the login application 134 on the mobile communication device 13 (step S10). In a subsequent step S11 the user U then is required to enter login information into the login application 134.

In a second portion S2 of the setup phase, an administrator A, by accessing settings of the host device 11, creates a user account in the host application 114 of the host device 11. Upon creation of the user account (step S20), the host device 11 starts searching for mobile communication devices 13 in its vicinity in order to pair up with a mobile communication device 13 using e.g. an NFC short range data connection. Once the mobile communication device 13 is brought into proximity with the communication module 112 of the host device 11, as it is shown in Fig. 3B, login information entered by the user U into the login application 134 of the communication device 13 is transferred to the host application 114 of the host device 11 (step S21) and is stored in a database of the host application 114 in connection with the user account of the user U.

The login application 134 of the mobile communication device 13 and the host application 114 of the host device 11 are now both configured to allow the user U to login to the host device 11 making use of his/her mobile communication device 13.

The login takes place, as illustrated in Fig. 4A and 4B, by placing the mobile communication device 13 close to the antenna of the communication module 112 of the host device 11 such that a data connection D1 is established in between the mobile communication device 13 and the host device 11, upon which login information is transmitted from the mobile communication device 13 to the host device 11 (step S30 during the regular login procedure S3). The host application 114 compares the received information with the information stored in connection with the user account, and if the information matches it allows the user U to access functions of the host device 11, in particular in order to control and/or monitor infusion operations of infusion devices connected to the host device 11.

The information may be transmitted from the mobile communication device 13 to the host device 11 in conventional NFC messages. The host application 114 herein parses the information from the NFC message and processes the information to identify and authorize the user.

The invention is not limited to the embodiments described above, but may be implemented in an entirely different fashion.

In particular, also a short range communication technology other than NFC may be used in order to exchange login information.

A host device may be used in connection with medical devices of different kinds and is not limited particularly to infusion devices.

The host device may be integrated into a medical device such as an infusion device, such that the host device and the medical device not necessarily represent separate entities. For example, the host device may be formed by circuitry and/or software installed in a medical device such as an infusion device.

By using his/her communication device, a user is enabled to automatically and comfortably log into a host device, in particular without having to type in login information into the host device. The login takes place automatically when approaching the mobile communication device to the host device, without further user interaction.

### List of Reference Numerals

- 1: Medical system
- 10: Medical devices (infusion devices)
- 100: Rack
- 11: Host device (infusion manager)
- 110: Processor
- 111: Storage unit
- 112: Communication module
- 114: Host application
- 12: Stand
- 13: Mobile communication device
- 130: Communication module
- 131: Storage device
- 132: Communication module
- 133: Processor
- 134: Login application
- 2: Outside communication network
- A: Administrator
- D1, D2: Communication connection
- P: Patient
- S1: First setup phase
- S10, S11: Steps
- S2: Second setup phase
- S20, S21: Steps
- S3: Login phase
- U: User

## Claims

1. Medical system (1), comprising:
- a host device (11) for monitoring and/or controlling an operation of at least one medical device (10) for performing a medical function, the host device (11) being configured to execute a host application (114), wherein functions of the host device (11) are accessible by a user (U) by providing login information to the host application (114), and
- a mobile communication device (13),
**characterized in**
**that** the mobile communication device (13) is configured to run a login application (134) which stores said login information, wherein the host device (11) and the mobile communication device (13) are configured to establish a wireless communication connection (D1) to transmit said login information from the mobile communication device (13) to the host device (11) to allow the user (U) to access said functions of the host device (11).

2. Medical system (1) according to claim 1, **characterized in that** the communication connection (D1) is a short-range wireless communication connection.

3. Medical system (1) according to claim 1 or 2, **characterized in that** the communication connection (D1) is an NFC connection, a Bluetooth connection, a WiFi connection, an Infrared connection or a Zigbee connection.

4. Medical system (1) according to one of claims 1 to 3, **characterized in that** the host device (11) and the mobile communication device (13) each comprise a communication module (112, 132) for establishing the wireless communication connection (D1).

5. Medical system (1) according to one of the preceding claims, **characterized in that** the wireless communication connection (D1) is a secure connection configured for an encrypted data transfer.

6. Medical system (1) according to one of the preceding claims, **characterized in that** the login application (134) stores, as login information, at least one of a first name of the user (U), a last name of the user (U), a login name of the user (U), a password of the user (U), a last login date, a last login method, identification information relating to the mobile communication device (13), and an encryption key.

7. Medical system (1) according to one of the preceding claims, **characterized in that** the host application (114) stores user account information relating to the user (U).

8. Medical system (1) according to claim 7, **characterized in that** the host application (114) is configured to evaluate the login information received from the mobile communication device (13) based on the stored user account information relating to the user (U) and to allow the user (U) to access said functions of the host device (11) based on the evaluation.

9. Method to operate a medical system (1), in which:
- a host device (11) for monitoring and/or controlling an operation of at least one medical device (10) for performing a medical function executes a host application (114), wherein functions of the host device (11) are accessible by a user (U) by providing login information to the host application (114),
**characterized in**
**that** a mobile communication device (13) runs a login application (134) which stores said login information, wherein, for the user (U) to gain access to said functions of the host device (11), a wireless communication connection (D1) is established between the host device (11) and the mobile communication device (13) to transmit said login information from the mobile communication device (13) to the host device (11).

10. Method according to claim 9, **characterized in that** in a setup phase a user (U) is requested to input information into the mobile communication device (13) to be stored in the login application (134) of the mobile communication device (13) as login information.

11. Method according to claim 10, **characterized in that** in the setup phase a user account is established in the host application (114) by transmitting login information relating to the user (U) from the mobile communication device (13) to the host device (11) using a wireless communication connection (D1), wherein the host application (114) stores said login information transmitted during the setup phase as user account information relating to the user (U).

12. Method according to claim 11, **characterized in that** during the setup phase the login information is transmitted using a non-secure connection.

13. Method according to one of claims 9 to 12, **characterized in that** software executed by the host device is automatically configured according to settings associated with the user upon a successful authentication of the user.
